(19) Europäisches Patentamt
European Patent Office
Office européen des brevets

(11) **EP 2 194 381 B1**

(12) **EUROPÄISCHE PATENTSCHRIFT**

(45) Veröffentlichungstag und Bekanntmachung des Hinweises auf die Patenterteilung:
**02.12.2015 Patentblatt 2015/49**

(51) Int Cl.:
***G01N 33/558*** *(2006.01)*

(21) Anmeldenummer: **08020964.6**

(22) Anmeldetag: **03.12.2008**

(54) **Testelement mit kombinierter Kontroll- und Kalibrationszone**

Testing element with combined control and calibration zone

Elément de test doté d'une zone de contrôle et de calibrage combinée

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(43) Veröffentlichungstag der Anmeldung:
**09.06.2010 Patentblatt 2010/23**

(73) Patentinhaber:
• **Roche Diagnostics GmbH**
 **68305 Mannheim (DE)**
 Benannte Vertragsstaaten:
 **DE**
• **F.Hoffmann-La Roche AG**
 **4070 Basel (CH)**
 Benannte Vertragsstaaten:
 **AT BE BG CH CY CZ DK EE ES FI FR GB GR HR HU IE IS IT LI LT LU LV MC MT NL NO PL PT RO SE SI SK TR**

(72) Erfinder: **Fischer, Thomas**
 **69231 Rauenberg (DE)**

(74) Vertreter: **Schwarz, Ralf et al**
 **Roche Diagnostics GmbH**
 **Patentabteilung,**
 **Sandhofer Strasse 116**
 **68305 Mannheim (DE)**

(56) Entgegenhaltungen:
**EP-A- 1 710 565**     **WO-A-97/09620**
**US-A1- 2005 112 780**     **US-A1- 2006 240 541**

**Beschreibung**

Die vorliegende Erfindung bezieht sich auf das technische Gebiet der Testelemente für die Analyse flüssiger Probenmaterialien oder von Probenmaterialien, die in flüssige Form überführt werden können.

Gegenstand der Erfindung ist ein Testelement, insbesondere in Form eines nach dem Sandwichprinzip arbeitenden immunologischen Teststreifens, für den fluorophoren Nachweis eines oder mehrerer Analyten in einer Probe, umfassend eine Analytnachweiszone, und eine kombinierte Kontroll- und Kalibrationszone, wobei die kombinierte Kontroll- und Kalibrationszone Fluorophor und Bindepartner für die spezifische Bindung von mit einem Fluorophor markierte Reagenzien umfasst.

Weiterhin betrifft die Erfindung ein Verfahren zur Kalibration eines Analyt-spezifischen Messsignals, ein Verfahren zur Bestimmung der Konzentration eines Analyten in einer Probe, und die Verwendung des Testelements zum Kalibrieren eines in der Analytnachweiszone eines Testelements erzeugten Signals.

Zum Nachweis von Analyten in einer Probe bzw. einem Probenmaterial werden häufig Testelemente verwendet, die eine oder mehrere diskrete Zonen mit immobilisierten Reaktanden aufweisen. Diese Zonen werden im Folgenden als Analytnachweiszonen, Nachweiszonen oder Detektionszonen bezeichnet. Durch eine spezifische Wechselwirkung mit den Reaktanden wird der Analyt in einer Reaktion ("Assay") aus der Probe in der Analytnachweiszone gebunden. Die Bindungsereignisse sind messtechnisch nachweisbar, z.B. durch optischen Nachweis von Fluoreszenzsignalen. Die Testelemente können mit Mikroarray-Strukturen präpariert sein und in Form eines Biochips für den ortsselektiven Nachweis von Bindungsreaktionen vorliegen, wie er beispielsweise in den publizierten Anmeldungen WO 2007/042219 und EP1412533 beschrieben wird. Bei den Testelementen kann es sich auch um Teststreifen handeln, auf denen beispielsweise eine oder mehrere Detektionszonen mit immobilisierten Reaktanden in Form von Streifen bzw. Strichen senkrecht zur Fließrichtung der Probe angeordnet sind. Solche Teststreifen werden beispielsweise beschrieben in der WO 2008/105814 und der US 2004-0126767.

Zum Nachweis der Bindungsereignisse zwischen den Reaktanden und dem Analyten in der Analytnachweiszone werden markierte Bindepartner eingesetzt. Es handelt sich dabei um Reaktanden, die ein Markierungsreagenz umfassen und spezifisch mit dem Analyt wechselwirken. Als Markierungsreagenzien können beispielsweise Fluorophore verwendet werden, die nach Anregung durch elektromagnetische Strahlung einer bestimmten Wellenlänge ein Lichtsignal abgeben. Die Messbarkeit dieses Lichtsignals in der Analytnachweiszone und seine Intensität weisen auf das Vorhandensein des Analyten in der Probe hin bzw. sind ein Maß für dessen Konzentration.

Bei immunologischen Nachweisverfahren beruhen die Bindungsereignisse auf dem Testelement auf einer spezifischen Antigen-Antikörper-Wechselwirkung ("Immunoassay"). Bei immunologischen Nachweisverfahren nach dem "Sandwich"-Prinzip wird typischerweise die zu analysierende Probe mit einem Konjugat aus einem Markierungsreagenz und einem Analyt-spezifischen Bindungspartner (z.B. einem Antikörper) in Kontakt gebracht, so dass Komplexe zwischen dem Analyt - sofern in der Probe vorhanden - und dem markierten Konjugat entstehen. Auf dem Testelement reagieren diese Komplexe mit den immobilisierten Reaktanden (z.B. Antikörpern), so dass in der Analytnachweiszone "Sandwich"-Komplexe aus dem Analyt/Konjugat und den immobilisierten Reaktanden vorliegen, die sich aufgrund der Markierung nachweisen lassen. Beispiele für solche Immunoassays werden beschrieben in den US-Patenten US 4,168,146 (Grubb et al.) und US 4,366,241 (Tom et al.). Eine alternative Technik ist der kompetitive Assay. Beim kompetitiven Assay ist das Markierungsreagenz im Allgemeinen konjugiert mit einem Bindepartner, der identisch ist mit dem Analyten oder ein Analytanalogon darstellt. Auf diese Weise kompetiert das markierte Konjugat mit dem eigentlichen Analyten um die Bindung an die Reaktanden in der Analytnachweiszone auf dem Testelement. Ein kompetitiver Assay wird üblicherweise zum Nachweis eines Antigens genutzt, wenn für dieses entweder nur ein einzelner spezifischer Antikörper zur Verfügung steht oder wenn das Antigen nicht über ausreichende Bindungsstellen für die ungehinderte Bindung von zwei Antikörpern verfügt. Diese Assay-Variante eignet sich damit auch zum Nachweis von Haptenen. Beispiele für kompetitive Assays werden beschrieben in den US-Patenten US 4,235,601 (Deutsch et al.), US 4,442,204 (Liotta) und US 5,208,535 (Buechler et al.).

Testelemente in Form immunologischer Teststreifen stellen ein weit verbreitetes Hilfsmittel zur schnellen Bestimmung von Drogen, Schwangerschaftshormonen, Infektionskrankheiten, oder so genannten "Cardiac Markem", wie Troponin T, dar. Dabei haben sowohl qualitative Tests, die rein visuell ausgelesen werden und oft nur eine "Ja-Nein"-Antwort liefern, als auch quantitative Tests, die mittels eines Auslesegerätes ausgewertet werden, breite Anwendung gefunden.

Schnelltests für immunologisch nachweisbare Substanzen sind für eine Vielzahl von unterschiedlichen Parametern seit langem bekannt, beispielsweise aus WO 97/06439, EP 0 291 194, US 5,591,645, US 4,861,711, US 5,141,850, US 6,506,612,

US 5,458,852, US 5,073,484. Hier werden meist die immunologischen Nachweisreagenzien (im Wesentlichen markierte und unmarkierte Antikörper bzw. Antigene) in trockener Form auf einem Träger bereitgestellt, der den Transport einer Probenflüssigkeit (insbesondere Körperflüssigkeiten wie Blut, Serum, Plasma, Urin, Speichel etc.) auf oder in dem Träger erlaubt. Vorzugsweise ist der Träger hierzu kapillaraktiv, beispielsweise eine Membran oder ein mit Kapillarkanälen versehener Kunststoffträger (wie z. B. in US 5,458,852). In der Fachwelt spricht man oft von immunologischen oder immunchromatographischen Teststreifen oder Testdevices. Diese Begriffe sowie der Ausdruck "trägergebundene immunologische Tests" oder "trägergebundene immunologische Testelemente" werden oftmals synonym verwendet und sollen auch im Folgenden austauschbar sein.

Neben einer oder mehrerer Analytnachweiszonen für den Nachweis von einem oder mehreren Analyten weisen die Testelemente üblicherweise eine weitere Zone auf, in der Reaktanden vorhanden sind, die den markierten Bindepartner spezifisch binden. Diese Zone wird als Kontrollzone oder Indikatorzone bezeichnet und dient als Funktionskontrolle für den markierten Bindepartner, indem sie den markierten Bindepartner abfängt, ohne dass eine direkte Beteiligung des Analyten erforderlich ist. Analytnachweiszone(n) und Kontrollzone sind üblicherweise räumlich eng begrenzt und deutlich voneinander getrennt auf dem Testelement angeordnet. Bei Testelementen in Form eines Teststreifens, bei dem die Probe in flüssiger Form aufgetragen und in die verschiedenen Zonen mit Hilfe Kapillarkräften gelangt, befindet sich die Kontrollzone als Kontrollstrich meist stromabwärts von der Analytnachweiszone auf oder in dem durchströmungsfähigen Material. Auf derartigen Teststreifen dient die Kontrollzone zudem als Laufkontrolle um sicher zu gehen, dass der markierte Bindepartner auch tatsächlich zu den einzelnen Zonen gelangt. Die Funktion der Kontrollzone als Kontrolle für die Funktionalität des markierten Bindepartners und als Laufkontrolle für die Funktionalität eines Teststreifens wird vom Fachmann auch als "Kontrollfunktion" bezeichnet.

In der US 2007/0048807 A1 wird ein Testelement beschrieben, das auf einer porösen Membran neben der Nachweiszone eine Indikatorzone aufweist. In der Indikatorzone ist ein Material immobilisiert ("receptive material"), das spezifisch mit Markierungsreagenz konjugierte Bindepartner bindet, die nicht mit dem Analyten in der Analytnachweiszone komplexiert sind.

Aufgrund der räumlichen Trennung von Analytnachweiszone und Kontrollzone befinden sich im Messgerät zur Auswertung der Testelemente für die Auswertung der Analytnachweis- und Kontrollzone oftmals ortsauflösende optische Systeme wie z. B. Kamerachips, oder 2- oder 3-dimensionale Photodiodenarrays. Die Signale der optischen Systeme werden dann von einer entsprechenden Auswertesoftware in Konzentrationswerte umgewandelt und zur Anzeige gebracht.

Ist das Markierungsreagenz des markierten Bindepartners ein Fluorophor, so ist neben der Kontrolle der Funktion des markierten Bindepartners durch eine Kontrollzone auch die Kalibrierung des Analyt-spezifischen Messsignals erforderlich. Bei dem Analyt-spezifischen Messsignal handelt es sich um das Signal, das, bedingt durch die Anwesenheit des Analyten in der Probe, in der Analytnachweiszone erzeugt werden kann. Die Intensität dieses Signals hängt von der eingestrahlten Energie im Anregungsspektrum des Fluorophors ab, der durch die Analyt-spezifische Wechselwirkung in der Analytnachweiszone lokalisiert ist. Die durch die Strahlungsquelle erzeugte Energie wiederum unterliegt erfahrungsgemäß Schwankungen, die einerseits auf herstellungsbedingte Variationen zwischen verschiedenen Strahlungsquellen des gleichen Typs zurückgeführt werden können oder sich andererseits über den Gebrauchszyklus einer Strahlungsquelle hinweg ergeben. Für eine reproduzierbare und verlässliche quantitative Bestimmung des Analyten müssen solche Schwankungen korrigiert werden. Dies erfolgt üblicherweise durch eine Kalibrierung des Analyt-spezifischen Messsignals mit einem Signal, das durch eine standardisierte Menge des Fluorophors erzeugt wird. Die standardisierte Menge des Fluorophors wird vom Fachmann auch als Fluorophor-Standard oder Kalibrationsstandard bezeichnet. Die nach Anregung mit elektromagnetischer Strahlung durch den Fluorophor-Standard emittierte Strahlung wird üblicherweise als "Kalibrationssignal" genannt.

Bei den im Stand der Technik verwendet Systemen zur (semi)quantitativen Bestimmung von Analyten kann die Kalibrierung des Analyt-spezifischen Messsignals durch Erzeugung des Kalibrationssignals außerhalb des Testelements durchgeführt werden (beispielsweise Triage®, Biosite Inc.).

Daneben gibt es Systeme, bei denen die Kalibration des Analyt-spezifischen Messsignals direkt mit dem Testelement durchgeführt wird. Solche Testelemente umfassen neben der Analytnachweiszone und ggf. der Kontrollzone noch eine weitere, von den anderen Zonen räumlich getrennte diskrete Zone, in der der Fluorophor als Markierungsreagenz in einer definierten Menge direkt auf dem Testelement angeordnet ist. Diese Zone wird im Folgenden als Kalibrationszone bezeichnet. Durch das Vorhandensein der Kalibrationszone auf dem Testelement kann das Kalibrierungssignal direkt auf dem Testelement mit dem gleichen Mess- oder Analysegerät detektiert werden, welches auch die Signale der Analytnachweiszone(n) und/oder der Kontrollzone erfasst. Im Falle eines Teststreifens oder Microarrays kann die Signalerfassung mit dem gleichen ortsauflösenden optischen System durchgeführt werden wie die Funktionskontrolle und die Messung des Analyt-abhängigen Signals in der Analytnachweiszone. Bei immunologischen Teststreifen kann die Kalibrationszone beispielsweise in Form eines weiteren Striches senkrecht zur Fließrichtung der Probe vorliegen (beispielsweise CARDIAC reader, Roche).

Das Vorhandensein einer Kalibrationszone auf dem Teststreifen hat den Vorteil, dass sich die Stabilitätsanforderung an das Fluorophor in dieser Zone auf die Lebensdauer der Charge des Testelements reduziert. Bei der Erzeugung des Kalibrationssignals außerhalb des Testelements hat der Anwender dagegen nicht nur darauf zu achten, dass die Haltbarkeit des Teststreifens gegeben ist, sondern auch die des separat verwendeten Fluorophor-Standards, wodurch jeweils eine zusätzliche Fehlerquelle vermieden werden muss. Weil die Generierung und Erfassung des Kalibrationssignals außerhalb des Testelements in der Regel zusätzliche Strahlungsquellen, Filter etc. und eine separate Auswerteoptik erfordert, sind die entsprechenden Analysegeräte aufwendiger konstruiert, größer und meistens auch teurer.

Im Zusammenhang mit der Formulierung "Kalibrierung des Analyt-spezifischen Messsignals" wird üblicherweise auch der Begriff "Kalibrationsfunktion" verwendet. Ebenso wie die Formulierung "Kalibrierung des Analyt-spezifischen Messsignals" ist der Begriff "Kalibrationsfunktion" dem Fachmann bekannt und bezieht sich auf Mittel, die für die Erzeugung des Kalibrationssignals beispielsweise auf einem Testträger oder - wie oben beschrieben - außerhalb eines Testträgers innerhalb eines Analysegerätes verwendet werden.

Mit Hilfe eines Testelements können auch mehrere, unterschiedliche Analyte in einer Probe gleichzeitig bestimmt werden. Bei solchen "Paneltests" ist für jeden Analyt auf dem Testelement eine diskrete Nachweiszone mit Analyt-spezifischen Reaktanden vorhanden. Da üblicherweise im Labor oder beim praktizierenden Arzt ein Analysegerät für die Auswertung verschiedener Testelemente zur Bestimmung unterschiedlicher medizinischer Parameter eingesetzt wird, müssen die entsprechenden Testelemente eine einheitliche, in Bezug auf das Gerät genormte Größe aufweisen. Aus diesem Grund steht mit steigender Zahl der Analytnachweiszonen auf dem Testelement immer weniger Platz für die Kontroll- und die Kalibrationszone zur Verfügung. Die Größe des Testelements selbst ist zudem durch das Erfordernis einer ausreichenden Benetzung der Testfläche durch die in der Regel kleinen Probenvolumina begrenzt. Da außerdem eine räumliche Trennung zwischen Kalibrations- und Analytnachweiszone gegeben sein muss, ist auch die Zahl der Analytnachweiszonen auf dem Testelement und somit die Anzahl der mit einem Testelement nachweisbaren Analyte begrenzt. Um dennoch im Rahmen eines Paneltests möglichst viele Analyte mit Hilfe eines Testträgers nachzuweisen, ist es daher oft notwendig, die für die Kalibration des Analyt-spezifischen Messsignals erforderlichen Messungen außerhalb des Testelements durchzuführen. Dies erfordert jedoch entsprechend aufwendige Anpassungen des Analyse- bzw. Messgerätes und/oder den Einsatz zusätzlicher Testelemente, und ist - aus den oben genannten Gründen - auch mit einem erhöhten Aufwand für das Laborpersonal verbunden.

Der vorliegenden Erfindung liegt die Aufgabe zugrunde, die Nachteile des Standes der Technik zu beseitigen. Insbesondere liegt der vorliegenden Erfindung die Aufgabe zugrunde, ein Testelement bereitzustellen, bei dem die Kalibration des Analyt-spezifischen Messsignals direkt mit dem Testelement durchgeführt werden kann und die Kontroll- und die Kalibrationszone auf dem Testelement so angeordnet ist, dass auch der Nachweis einer größeren Zahl von Analyten mit Hilfe des Testelements möglich ist.

Diese Aufgabe wird durch den Gegenstand der Erfindung gelöst.

Gegenstand der Erfindung ist ein Testelement gemäß Anspruch 1, ein Verfahren zur Kalibration eines Analyt-spezifischen Messsignals gemäß Anspruch 7, ein Verfahren zur Bestimmung der Konzentration eines Analyten in einer Probe gemäß Anspruch 8, und eine Verwendung gemäß Anspruch 12. Bevorzugte Ausführungsformen der Erfindung sind Gegenstand der abhängigen Ansprüche.

Die Erfindung ermöglicht bei Testelementen, bei denen der spezifische Nachweis eines oder mehrerer Analyten in einer Probe mit Hilfe von Fluorophoren erfolgt, die Erfassung des Signals für die Kontrollfunktion und des Signals für die Kalibrationsfunktion in *einer* diskreten, räumlich begrenzten Zone. Erfindungsgemäß wird vorgeschlagen, in einem Testelement neben der üblichen Analytnachweiszone eine weitere Zone anzuordnen. Diese Zone, die im Folgenden als kombinierte Kontroll- und Kalibrationszone bezeichnet wird, liegt im Testelement räumlich getrennt von der Analytnachweiszone vor, und sie umfasst Fluorophor sowie Bindepartner für die spezifische Bindung von mit einem Fluorophor markierten Reagenzien. Die räumliche Trennung von kombinierter Kontroll- und Kalibrationszone und Analytnachweiszone erfolgt in einer Weise, dass Analytnachweiszone und kombinierte Kontroll- und Kalibrationszone jeweils als diskrete, räumlich begrenzte Zonen vorliegen.

Gegenstand der vorliegenden Erfindung ist somit ein Testelement für den fluorophoren Nachweis eines oder mehrerer Analyten in einer Probe, umfassend

- eine Analytnachweiszone, und
- eine kombinierte Kontroll- und Kalibrationszone,
  dadurch gekennzeichnet, dass
  die kombinierte Kontroll- und Kalibrationszone

    a) Fluorophor, wobei der auf der kombinierten Kalibrations-/Kontrollzone gebundene Fluorophor als Kalibrationsstandard dient und
    b) Bindepartner für die spezifische Bindung von mit einem Fluorophor markierten Reagenzien umfasst, wobei die Bindepartner für die spezifische Bindung von mit Fluorophor markierten Reagenzien immobilisiert vorliegen, wobei die mit einem Fluorophor markierten Reagenzien mit dem Analyten spezifisch durch direkte oder indirekte Bindung wechselwirken

Geeignete Fluorophore sind dem Fachmann bekannt. In einer bevorzugten Ausführungsform handelt es sich es sich bei dem Fluorophor des Fluorophors in der kombinierten Kontroll- und Kalibrationszone und/oder des Fluorophors der mit dem Fluorophor markierten Reagenzien um einen Fluoreszenzfarbstoff bzw. um ein Fluoreszenzlabel. Die Begriffe Fluoreszenzfarbstoff, Fluoreszenzlabel oder Fluoreszenzmarkierung werden im folgenden synonym verwendet. Typische und für die Erfindung geeigneten Fluoreszenzlabel können beispielsweise Fluoreszenzproteine (wie GFP und seine Derivate), Cy3, Cy5, Texas Red, Fluorescein, und die Alexa-Farbstoffe (z.B. Alexa 568) sein. Weitere Fluoreszenzlabel können beispielsweise bei der Firma Invitrogen erhältlich.

Bei dem mit dem Fluorophor markierten Reagenzien handelt es sich um einen Bindepartner, der mit dem Fluorophor markiert ist, so dass über die Markierung ein Nachweis des Bindepartners möglich ist. Die mit dem Fluorophor markierten Reagenzien sind in der Lage, mit dem Analyten spezifisch zu wechselwirken. Diese Wechselwirkung kann in Form einer direkten oder indirekten Bindung erfolgen. Wesentlich ist, dass aufgrund der spezifischen Wechselwirkung der mit dem Fluorophor markierten Reagenzien mit dem Analyten über die Markierung ein Nachweis des Analyten möglich ist.

Die durch das Erfordernis einer ausreichenden Benetzung der Testfläche durch die in der Regel kleinen Probenvolumina begrenzte Fläche des Testelements und die notwendige räumliche Trennung von Kalibrations-, Kontroll- und Analytnachweiszone(n) limitiert die Anzahl der Analytnachweiszonen auf einem Testelement. Die erfindungsgemäße kombinierte Kontroll- und Kalibrationszone ermöglicht mindestens eine zusätzliche Analytnachweiszone auf dem Testelement, ohne dass der Anwender auf ein zusätzliches Testelement zurückgreifen oder auf ein entsprechend aufwendigeres analytisches System ausweichen müsste, welches eine Kalibrierung des Analyt-spezifischen Messsignals nur mit Hilfe eines Fluorophor-Standards außerhalb des Testelements erlaubt.

In einer Ausführungsform der Erfindung handelt es sich bei dem Testelement um einen Biochip, in dem die Analytnachweiszone(n) und die kombinierte Kontroll- und Kalibrationszone in Form eines Microarrays angeordnet sind. Solche Arrays, zu denen "Gen-Chips", "Protein-Chips", Antikörper-Arrays o. ä. zählen, sind dem Fachmann bekannt und werden üblicherweise aus mit Polykationen, Nitrozellulose oder Biotin-beschichteten Glas-Trägern, Deckgläschen, oder aus Membranen, beispielsweise Nitrozellulose- oder Nylon-Membranen hergestellt.

In einer bevorzugten Ausführungsform handelt es sich bei dem Testelement um einen Teststreifen, bei dem die Analytnachweiszone und die kombinierte Kontroll- und Kalibrationszone so angeordnet sind, dass ein Flüssigkeitstransport zwischen den Zonen möglich ist. Hierzu enthält der Teststreifen typischerweise meist ein durchströmungsfähiges Material (z. B. Papier, Vlies, Membran, Kapillarkanal) auf, das ggf. auf einem inerten Träger befestigt ist.

Typischerweise weist ein Testelement je eine oder mehrere Probenaufgabezonen, Saugzonen, Chromatographiezonen, Nachweiszonen, Reaktionszonen, Kontrollzonen und/oder Kalibrationszonen auf. Wichtig für die Erfindung ist lediglich, dass zumindest eine (Analyt-)Nachweiszone und zumindest eine kombinierte Kontroll- und Kalibrationszone vorhanden ist.

In einer Ausführungsform der Erfindung liegen die Analytnachweiszone und/oder die kombinierte Kontroll- und Kalibrationszone in Form eines Striches oder im Wesentlichen runden Spots vor.

Als Testelement im Sinne der Erfindung sind alle Testelemente geeignet, die auf spezifischen Bindereaktionen basieren und bei denen der Nachweis der spezifischen Bindereaktion mit Hilfe eines von einem Fluorophor erzeugten Signals erfolgt. Entsprechend spezifische Bindereaktionen sind dem Fachmann bekannt. Beispielhaft genannt seien die Bindepaare:

Antikörper mit Hapten, Antigen oder anderen Antikörpern (beispielsweise speziesspezifische Antikörper-Antikörper-Wechselwirkungen), wobei teilweise auch jeweils Fragmente dieser Spezies genügen;
Biotin mit Avidin oder Streptavidin;
Hormon mit Hormonrezeptor;
Zucker mit Lektin;
Nukleinsäure mit komplementärer Nukleinsäure; und dergleichen mehr.

In einer besonders bevorzugten Ausführungsform handelt es sich bei dem Testelement um ein Testelement in Form eines nach dem Sandwichprinzip arbeitenden immunologischen Teststreifens.

Zur Lösung der der Erfindung zugrunde liegenden Aufgabe ist es unerheblich, ob der Fluorophor in der kombinierten Kontroll- und Kalibrationszone und der Fluorophor der mit dem Fluorophor markierten Reagenzien bei unterschiedlichen Wellenlängen und/oder in verschiedenen Spektralbereichen emittieren. Somit ist es im Sinne der Erfindung auch nicht notwendig, dass der Fluorophor in der kombinierten Kontroll- und Kalibrationszone und der Fluorophor der mit dem Fluorophor markierten Reagenzien von der Struktur her identisch sind.

In einer bevorzugten Ausführungsform der Erfindung weisen der Fluorophor in der kombinierten Kontroll- und Kalibrationszone und der Fluorophor der mit dem Fluorophor markierten Reagenzien das gleiche Strukturelement auf. Solche, für Fluorophore charakteristische Strukturelemente sind dem Fachmann bekannt. In einer anderen bevorzugten Ausführungsform sind der Fluorophor in der kombinierten Kontroll- und Kalibrationszone und der Fluorophor der mit dem Fluorophor markierten Reagenzien identisch.

Besonders bevorzugt ist eine Ausführungsform der vorliegenden Erfindung, in der der Fluorophor in der kombinierten Kontroll- und Kalibrationszone und der Fluorophor der mit dem Fluorophor markierten Reagenzien mit der im Wesentlichen gleichen Wellenlänge angeregt werden können und, als Folge dieser Anregung, elektromagnetische Strahlung der im Wesentlichen gleichen Wellenlänge emittieren. Der Vorteil dieser Ausführungsform liegt darin, dass für die Anregung und die Detektion des emittierten Signals des Fluorophors in der kombinierten Kontroll- und Kalibrationszone und des Fluorophors der mit dem Fluorophor markierten Reagenzien mit den jeweils gleichen Komponenten (Strahlungsquelle, Wellenlängenfilter etc.) eingesetzt werden kann und dadurch aufwendigere konstruktivere Anpassungen der in der Regel kleinen und kompakten Laborgeräte vermieden wird.

Die Markierung der mit dem Fluorophor markierten Reagenzien erfolgt direkt oder indirekt nach Methoden, die dem Fachmann bekannt sind. Bei der direkten Markierung wird der Fluorophor direkt (kovalent oder nicht kovalent) an den Bindepartner gekoppelt. Bei der indirekten Markierung wird beispielsweise ein sekundärer Bindepartner, der die Markierung trägt, (kovalent oder nicht kovalent) an den Analyt-spezifischen Bindepartner gebunden. Geeignete sekundäre oder auch tertiäre Bindepartner können Antikörper, Sekundär-Antikörper oder das bekannte Streptavidin/Biotin-System sein.

In der kombinierten Kontroll- und Kalibrationszone müssen das Fluorophor und die Bindepartner für die spezifische Bindung von mit einem Fluorophor markierten Reagenzien in einer Art und Weise vorliegen, dass zwei Voraussetzungen erfüllt sind:

1. In Abwesenheit der mit einem Fluorophor markierten Reagenzien kann durch spezifische Anregung des Fluorophors in der kombinierten Kontroll- und Kalibrationszone ein detektierbares Signal erzeugt werden, und

2. die Anwesenheit der mit einem Fluorophor markierten Reagenzien in der kombinierten Kontroll- und Kalibrationszone ermöglicht ein detektierbares Signal, das ein Maß für die Anwesenheit der mit einem Fluorophor markierten Reagenzien in der Zone darstellt.

Damit diese Voraussetzungen erfüllt sind, werden das Fluorophor und die Bindepartner für die spezifische Bindung der mit einem Fluorophor markierten Reagenzien in der kombinierten Kontroll- und Kalibrationszone in einer bestimmten Konzentration und Menge innerhalb eines bestimmten Toleranzbereiches eingesetzt. Der Toleranzbereich muss so gewählt werden, dass sowohl die Kontroll- als auch die Kalibrationsfunktion genutzt werden kann und das Signal der Kalibrationsfunktion, d.h. das durch das Fluorophor in der kombinierten Kontroll- und Kalibrationszone erzeugte Signal keinen Einfluss auf die Intensität das Signals der Kontrollfunktion, d.h. des Signals der mit einem Fluorophor markierten Reagenzien, hat.

Vorzugsweise liegt in der kombinierten Kontroll- und Kalibrationszone des erfindungsgemäßen Testelements die Menge des Fluorophors zwischen 0,001 $\mu$g und 5 $\mu$g, während die Menge der Bindepartner für die spezifische Bindung der mit einem Fluorophor markierten Reagenzien zwischen 1ng und 500ng beträgt. Besonders bevorzugt ist in der kombinierten Kontroll- und Kalibrationszone eine Menge des Fluorophors zwischen 0,1$\mu$g und 1$\mu$g, und eine Menge der Bindepartner für die spezifische Bindung der mit einem Fluorophor markierten Reagenzien zwischen 10ng und 50ng.

Eine bevorzugte Ausführungsform des erfindungsgemäßen Testelements umfasst eine kombinierte Kontroll- und Kalibrationszone, die durch Imprägnierung des Testelements erzeugt wurde, **dadurch gekennzeichnet, dass** bei der Imprägnierung Fluorophor in einer Lösung mit einer Konzentration zwischen 0,25 nmol/ml und 2,5 nmol/ml und die mit einem Fluorophor markierten Reagenzien in einer Lösung mit einer Konzentration zwischen 0,01mg/ml und 5mg/ml eingesetzt wurde. In einer besonders bevorzugten Ausführungsform des Testelements wurde die kombinierte Kontroll- und Kalibrationszone durch Imprägnierung des Testelements mit dem gelösten Fluorophor in einer Konzentration zwischen 1 nmol/ml und 2 nmol/ml und mit den gelösten mit einem Fluorophor markierten Reagenzien in einer Konzentration zwischen 0,1 mg/ml und 1 mg/ml erzeugt.

Die Imprägnierung ist ein übliches Verfahren, mit dem für den Test erforderliche Komponenten auf Testelemente aufgebracht werden, beispielsweise in Form eines Striches auf einem immunologischen Teststreifen. Die bei der Imprägnierung einzusetzende Menge der sich in Lösung befindlichen Komponenten richtet sich nach dem Material des jeweiligen Testelements und ist dem Fachmann bekannt.

In einer bevorzugten Ausführungsform wird die Menge oder Konzentration des Fluorophors und der Bindepartner für die spezifische Bindung der mit einem Fluorophor markierten Reagenzien in der kombinierten Kontroll- und Kalibrationszone so gewählt, dass das Signal der Kontrollfunktion stärker ist als das Signal der Kalibrationsfunktion. Das heißt, in der kombinierten Kontroll- und Kalibrationszone des erfindungsgemäßen Testelements wird die Menge oder Konzentration des Fluorophors und der Bindepartner für die spezifische Bindung der mit einem Fluorophor markierten Reagenzien so gewählt, dass die Anzahl der Fluorophore kleiner ist als die Zahl der mit einem Fluorophor markierten Reagenzien nach Bindung an die Bindepartner für die spezifische Bindung der mit einem Fluorophor markierten Reagenzien.

Der Fachmann ist in der Lage, diese Werte für die jeweiligen Fluorophore und die Bindepartner für die spezifische Bindung der mit einem Fluorophor markierten Reagenzien zu bestimmen und den entsprechenden Toleranzbereich zu definieren, der üblicherweise bestimmte Variationen bezüglich des verwendeten Testträgers und des bzw. der eingesetzten Fluorophore aufweisen kann.

In einer bevorzugten Ausführungsform des erfindungsgemäßen Testelements sind die Bindepartner für die spezifische Bindung von mit dem Fluorophor markierten Reagenzien und/oder das Fluorophor in der kombinierten Kontroll- und Kalibrationszone immobilisiert. Die Immobilisierung kann in einer Art und Weise erfolgen, die dem Fachmann bekannt ist. Die Immobilisierung kann auf kovalenten Bindungen oder auf nicht kovalenten Wechselwirkungen beruhen, wie sie beispielsweise für das Streptavidin/Biotin-Interaktionssystem charakteristisch sind.

Das Fluorophor und die Bindepartner für die spezifische Bindung von mit einem Fluorophor markierte Reagenzien können in der kombinierten Kontroll- und Kalibrationszone direkt an das Testelement immobilisiert vorliegen oder indirekt durch Bindung an geeignete Trägermoleküle oder Bindestrukturen im Testelement. Die Trägermoleküle können beispielsweise spezifische Bindepartner für das Fluorophor und/oder für die Bindepartner für die spezifische Bindung von mit einem Fluorophor markierten Reagenzien sein, so dass die Immobilisierung nicht-kovalent aufgrund der spezifischen Wechselwirkung mit den Trägermolekül erfolgt. Das Trägermolekül selbst wiederum kann direkt oder indirekt über weitere Bindepartner/Trägermoleküle oder geeignete Bindestrukturen an das Testelement immobilisiert sein.

Für die Erfindung ist es unerheblich, ob die Lokalisierung des Fluorophors und/oder der Bindepartner für die spezifische Bindung von mit einem Fluorophor markierten Reagenzien bereits mit der Herstellung des Testelements in der kombinierten Kontroll- und Kalibrationszone erfolgt, oder erst kurz vor der Kalibration des Messsignals, beispielsweise indem das Fluorophor und/oder die Bindepartner für die spezifische Bindung von mit einem Fluorophor markierten Reagenzien zuvor in gelöster Form in einem geeigneten Lösungsmittel mit dem Testelement unter Bedingungen in Kontakt gebracht werden, die eine Immobilisierung in der kombinierten Kontroll- und Kalibrationszone ermöglichen.

Erfindungsgemäß geeignete Analyten sind Analyten, die sich aufgrund einer spezifischen Bindepaarbeziehung nachweisen lassen. Insbesondere für den bevorzugten Fall des Testelements in Form eines nach dem Sandwichprinzip arbeitenden immunologischen Teststreifens sind dies Antikörper, Antigene, Haptene (jeweils inkl. Fragmenten hiervon). Besonders bevorzugt sind die immunologisch nachweisbaren Analyten hCG, BNP, (NT-)proBNP, Troponin I, Troponin T, Myoglobin, D-Dimer, CRP, HIV, HCV, CD40, CK-MB, TSH, etc.

Als Probe, aus der die Analyten bestimmt werden können, sind erfindungsgemäß alle flüssigen oder in flüssige Form überführbaren Probenmaterialien geeignet. Insbesondere sind Körperflüssigkeiten wie Blut und daraus abgeleitete Fraktionen (Serum, Plasma), Speichel, Urin, zerebrospinale Flüssigkeit, Sperma, interstitielle Flüssigkeit, Schweiß und dergleichen mehr geeignet. Geeignet sind auch an sich nicht flüssige, jedoch durch Lösen oder Suspendieren in Lösungsmitteln, insbesondere wässrigen Lösemitteln, in die flüssige Phase überführbaren Probenmaterialien.

Die im Testelement enthaltenen oder zum Testelement bzw. zur Probe hinzuzufügenden Reaktanden (synonym auch als "Bindepartner" oder "spezifische Bindepartner" bezeichnet) gehen eine selektive (Binde-)Reaktion mit dem Analyten oder - im Falle der Bindepartner für die spezifische Bindung von mit einem Fluorophor markierten Reagenzien - mit den mit einem Fluorophor markierten Reagenzien ein. Sie lassen direkt oder indirekt Rückschluss auf die in der Probe vorhandene Analytmenge zu.

Bevorzugte Bindepartner sind Antikörper (AK; im Englischen antibodies oder AB), insbesondere polyklonale Antikörper (PAK; im Englischen polyclonal antibodies oder PAB) oder monoklonale Antikörper (MAK; im Englischen monoclonal antibodies oder MAB), sowie Antigene und Haptene, sowie Fragmente hiervon, sofern sie für die Zwecke des spezifischen Analytnachweises aktiv sind.

Vorzugsweise wird ein Teil der Reaktanden oder Reagenzien so auf dem Testelement zur Verfügung gestellt, dass sie durch die Probenflüssigkeit von diesem abgelöst werden können, beispielsweise durch Imprägnierung geeigneter Trägermaterialien wie Vliese, Membranen etc., oder durch Aufbringen und Trocknen in entsprechende (Kapillar-)Kanalstrukturen.

Es ist jedoch auch möglich, zumindest einen Reaktanden oder eines der Reagenzien in gelöster Form zum Testelement zuzugeben, beispielsweise indem die Probe mit der Lösung versetzt wird oder die Lösung unabhängig von der Probe auf das Testelement aufgebracht wird. Erfindungsgemäß ist es auch möglich, wenn auch weniger bevorzugt, alle spezifischen Reaktanden und/oder Reagenzien in einer Lösung oder in mehreren Lösungen zur Analyse einzusetzen. Auf dem Testelement befindet sich dann in der Analytnachweiszone lediglich ein weiterer Reaktand, der als Bindepartner einen spezifisch an den Analyt gebundenen Reaktanden, der nicht das mit dem Fluorophor markierte Reagenz ist, als spezifischen Bindepartner abfangen kann und so eine indirekte Bindung des Analyten in der Analytnachweiszone des Testelements bewirkt. Analog befindet sich in der Kontrollzone ein Bindepartner, der das mit dem Fluorophor markierte Reagenz abfangen kann, ohne dass eine direkte Beteiligung des Analyten erforderlich ist.

Der in der kombinierten Kontroll- und Kalibrationszone vorzugsweise immobilisierte Bindepartner für die spezifische Bindung der mit einem Fluorophor markierten Reagenzien ist in der Lage, die mit einem Fluorophor markierten Reagenzien spezifisch zu binden. Der Bindepartner kann beispielsweise ein Analytanalogon sein, oder - im Falle einer kompetitiven Testführung - ein Antikörper, der gegen bestimmte Epitope auf den mit einem Fluorophor markierten Reagenzien gerichtet ist, die nicht Analyt-spezifisch sind. Wichtig ist, dass der Bindepartner für die spezifische Bindung der mit einem Fluorophor markierten Reagenzien die mit einem Fluorophor markierten Reagenzien in der kombinierten Kontroll- und Kalibrationszone abfangen kann, ohne dass eine direkte Beteiligung des Analyten erforderlich ist.

Die Erfindung umfasst auch ein Verfahren zur Kalibration eines Analyt-spezifischen Messsignals unter Verwendung des erfindungsgemäßen Testelements und eines für die Auswertung des erfindungsgemäßen Testelements geeigneten Analysegeräts, umfassend die folgenden Schritte:

a) Erzeugung eines Signals in der kombinierten Kontroll- und Kalibrationszone durch Anregung des Fluorophors in der kombinierten Kontroll- und Kalibrationszone in Abwesenheit der mit einem Fluorophor markierten Reagenzien,

b) Messung des in Schritt a) in der kombinierten Kontroll- und Kalibrationszone erzeugten Signals,

c) in Kontakt bringen der Probe mit dem Testelement und mit den mit einem Fluorophor markierten Reagenzien, so dass der der Analyt - sofern in der Probe vorhanden - zu einem in der Analytnachweiszone detektierbaren Signal führt;

d) Erzeugung eines Signals in der Analynachweiszone durch Anregung des Fluorophors der in Anwesenheit des Analyten in der Analytnachweiszone vorhandenen mit einem Fluorophor markierten Reagenzien,

e) Messung des in Schritt d) in der Analytnachweiszone erzeugten Signals,

f) In Beziehung setzen der in Schritt b) und e) gemessenen Signale.

Für das erfindungsgemäße Verfahren wird neben dem erfindungsgemäßen

Testelement ein für die Auswertung des Testelements geeignetes Analysegerät bereit gestellt. Hierzu geeignete Geräte bzw. Analysegeräte sind dem Fachmann aus dem Stand der Technik bekannt. Dazu gehören beispielsweise Imaging / Scanning-Detektionssysteme, die die Messung bzw. Auswertung von Analyt-spezifischen Signalen aus der Analytnachweiszone und Signalen aus Kontroll- bzw. Kalibrationszone in einem Gerät ermöglichen. Erfindungsgemäß wichtig ist aber, dass sowohl das durch die Fluorophore in der Analytnachweiszone als auch das durch die Fluorophore in der kombinierten Kontroll- und Kalibrationszone erzeugte Signal vom Analysegerät erfasst werden. Als typisches Analyse- bzw. Messgerät sei das Triage® Analysegerät der Firma Biosite genannt.

Bei den im erfindungsgemäßen Verfahren erzeugten Signalen handelt es sich um elektromagnetische Strahlung, die nach Anregung des Fluorophors emittiert wird. Die Anregung erfolgt üblicherweise durch gezielte Bestrahlung des Fluorophors mit elektromagnetischer Strahlung einer für das betreffende Fluorophor charakteristischen (Anregungs-)Wellenlänge. Ob die Anregung mit elektromagnetischer Strahlung einer Wellenlänge erfolgt, die der maximalen Anregung entspricht oder mit elektromagnetischer Strahlung einer anderen Wellenlänge, ist unerheblich, solange die Bestrahlung zur Emission eines detektierbaren Signals führt, dessen Intensität ein Maß für die Menge des angeregten Fluorophors ist.

Die Probe wird mit dem Testelement und mit für den Analyten spezifischen Reagenzien in Kontakt gebracht und der Analyt führt - sofern er in der Probe vorhanden ist - durch Wechselwirkung mit den spezifischen Reagenzien zu einem in der Analytnachweiszone detektierbaren Signal, das auf die mit einem Fluorophor markierten Reagenzien zurückzuführen ist. Wichtig ist, dass die Intensität des Messsignals von der Menge an Analyt in der Probe abhängt.

Zur Kalibrierung des Analyt-spezifischen Messsignals müssen die in der kombinierten Kontroll- und Kalibrationszone und in der Analytnachweiszone gemessenen Signale (Schritt b) und e) des erfindungsgemäßen Verfahrens) zueinander in Beziehung gesetzt werden. Das in Schritt b) in der kombinierten Kontroll- und Kalibrationszone gemessene Signal dient dabei als Referenz für das eigentliche, Analyt-abhängige Signal, das in der Analytnachweiszone erzeugt wird. In einer bevorzugten Ausführungsform des erfindungsgemäßen Verfahrens werden die in der kombinierten Kontroll- und Kalibrationszone und in der Analytnachweiszone gemessenen Signale miteinander verrechnet. In einer besonders bevorzugten Ausführungsform des erfindungsgemäßen Verfahrens werden diese beiden Signale nach folgender Formel verrechnet:

$$\text{Signal (kalibriert)} = \text{Signal (Analytnachweiszone)} / \text{Signal (Kontroll- und Kalibrationszone)}.$$

"Signal (Analytnachweiszone)" entspricht dabei dem in Schritt d) des erfindungsgemäßen Verfahrens gemessenen Signal und "Signal (Kontroll- und Kalibrationszone)" entspricht dem in Schritt b) des erfindungsgemäßen Verfahrens gemessenen Signal.

Die Verrechnung der in Schritt b) und e) des erfindungsgemäßen Verfahrens erfassten Signale aus der Analytnachweiszone und der kombinierten Kontroll- und Kalibrationszone kann beispielsweise in einer zentralen Rechnereinheit des Analysegerätes erfolgen.

Das Ergebnis der Verrechnung der in Schritt b) und e) des erfindungsgemäßen Verfahrens erfassten Signale aus der Analytnachweiszone und der kombinierten Kontroll- und Kalibrationszone kann zur Bestimmung der Analysekonzentration beispielsweise mit einer Eichkurve verglichen werden. Somit umfasst die Erfindung auch ein Verfahren zur Bestimmung der Konzentration eines Analyten in einer Probe unter Verwendung des erfindungsgemäßen Testelements und eines für die Auswertung des erfindungsgemäßen Testelements geeigneten Analysegeräts, umfassend die folgenden Schritte:

a) Erzeugung eines Signals in der kombinierten Kontroll- und Kalibrationszone durch Anregung des Fluorophors in der kombinierten Kontroll- und Kalibrationszone in Abwesenheit der mit einem Fluorophor markierten Reagenzien,

b) Messung des in Schritt a) in der kombinierten Kontroll- und Kalibrationszone erzeugten Signals,

c) in Kontakt bringen der Probe mit dem Testelement und mit den mit einem Fluorophor markierten Reagenzien, so dass der der Analyt - sofern in der Probe vorhanden - zu einem in der Analytnachweiszone detektierbaren Signal führt;

d) Erzeugung eines Signals in der Analytnachweiszone durch Anregung des Fluorophors der in Anwesenheit des Analyten in der Analytnachweiszone vorhandenen mit einem Fluorophor markierten Reagenzien,

e) Messung des in Schritt d) in der Analytnachweiszone erzeugten Signals,

f) In Beziehung setzen der in Schritt b) und e) gemessenen Signale.

g) Vergleich der Beziehung aus Schritt f) mit einer Eichkurve,

h) Bestimmung der Konzentration des Analyten aufgrund des in Schritt g) erfolgten Vergleiches.

Die im erfindungsgemäßen Verfahren verwendete Eichkurve wird durch das Vermessen von Standardlösungen mit bekannten Analytmengen erhalten; vorzugsweise erfolgt dabei jeweils eine Kalibration des Analyt-spezifischen Messsignals nach dem erfindungsgemäßen Verfahren zur Kalibration Analytspezifischer Messsignale.

Für die erfindungsgemäßen Verfahren ist es unerheblich, ob die Erzeugung und Messung des Signals in der kombinierten Kontroll- und Kalibrationszone durch Anregung des Fluorophors in der kombinierten Kontroll- und Kalibrationszone (Schritt a) und b) des erfindungsgemäßen Verfahrens) vor oder nach dem In Kontakt bringen der Probe mit dem Testelement erfolgt. Wesentlich ist, dass das durch Anregung des in der kombinierten Kontroll- und Kalibrationszone immobilisierten Fluorophors erzeugte Signal in Abwesenheit der mit einem Fluorophor markierten Reagenzien, die durch die in der kombinierten Kontroll- und Kalibrationszone immobilisierten Bindepartner spezifisch gebunden werden können, erfolgt.

Eine Ausfilhrungsform der erfindungsgemäßen Verfahren ist dadurch **gekennzeichnet, dass** die Erzeugung des Signals in der kombinierten Kontroll- und Kalibrationszone durch Anregung des Fluorophors in der kombinierten Kontroll- und Kalibrationszone in Abwesenheit der mit einem Fluorophor markierten Reagenzien (Schritt a)) und die Messung des durch den Fluorophor erzeugten Signals (Schritt b)) erfolgen, nachdem die Probe mit dem Testelement und mit den mit einem Fluorophor markierten Reagenzien in Kontakt gebracht wird (Schritt c)). So könnte die Probe beispielsweise vor dem Einbringen des Testträgers in das Analysegerät mit dem Testträger und den mit einem Fluorophor markierten Reagenzien in Kontakt gebracht werden. Die Erzeugung und Messung des Kalibrationssignals erfolgt dann bevor die mit einem Fluorophor markierten Reagenzien auf dem Testträger die kombinierte Kontroll- und Kalibrationszone - beispielsweise mit Hilfe von Kapillarkräften - erreicht haben. Die Probe kann auch vor dem Erzeugen und Messen des Kalibrationssignals mit dem Testträger in Kontakt gebracht werden, ohne dass die Probe mit den mit einem Fluorophor markierten Reagenzien in Kontakt kommt, beispielsweise weil die mit einem Fluorophor markierten Reagenzien nicht auf dem Testträger vorhanden sind und erst nach der Erfassung des Kalibrationssignals mit der Probe in Kontakt gebracht werden.

Es ist auch möglich, dass die Probe zuerst mit dem Testelement in Kontakt gebracht wird und die Messung des Signals in der Analytnachweiszone erfolgt (entsprechend Schritt e) der erfindungsgemäßen Verfahrens). Falls die mit einem Fluorophor markierten Reagenzien dann auf dem Testträger die kombinierte Kontroll- und Kalibrationszone - beispielsweise mit Hilfe von Kapillarkräften - erreicht haben, müssen die mit einem Fluorophor markierten Reagenzien, die durch die in der kombinierten Kontroll- und Kalibrationszone immobilisierten Bindepartner spezifisch gebunden wurden, von dem Testträger entfernt werden, beispielsweise durch einen oder mehrere Wasch-Schritte. Erst dann dürfen die für die Kalibration des Analyt-spezifischen Messsignals zusätzlich erforderliche Erzeugung und Messung des Kalibrationssignals in der kombinierten Kontroll- und Kalibrationszone durchgeführt werden (entsprechend Schritt a) und b) der erfindungsgemäßen Verfahrens).

In einer bevorzugten Ausführungsform der erfindungsgemäßen Verfahren erfolgt die Erzeugung des Signals in der kombinierten Kontroll- und Kalibrationszone durch Anregung des Fluorophors in der kombinierten Kontroll- und Kalibrationszone in Abwesenheit der mit einem Fluorophor markierten Reagenzien (Schritt a)) und die Messung des durch den Fluorophor erzeugten Signals (Schritt b)) bevor die Probe mit dem Testelement und mit den mit einem Fluorophor markierten Reagenzien in Kontakt gebracht wurde (Schritt c)).

Gegenstand der Erfindung ist auch die Verwendung des erfindungsgemäßen Testelements zum Kalibrieren eines in der Analytnachweiszone eines Testelements erzeugten Signals. In einer Ausführungsform der erfindungsgemäßen Verwendung werden die Analyt-spezifischen Signale mit Hilfe eines anderen Testelements erzeugt.

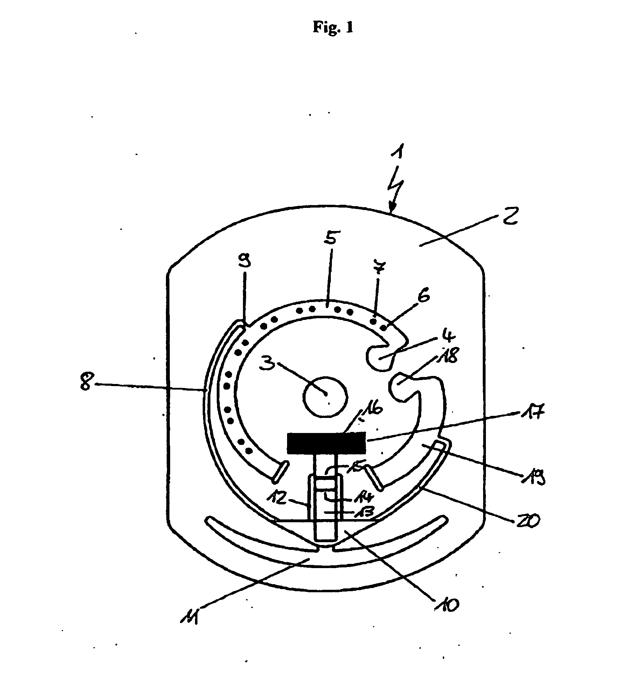

Fig. 1 zeigt die schematische Darstellung einer Aufsicht einer Ausführungsform des erfindungsgemäßen Testelements (1) mit einer Analytnachweiszone (14) und einer kombinierten Kontroll- und Kalibrationszone (15).

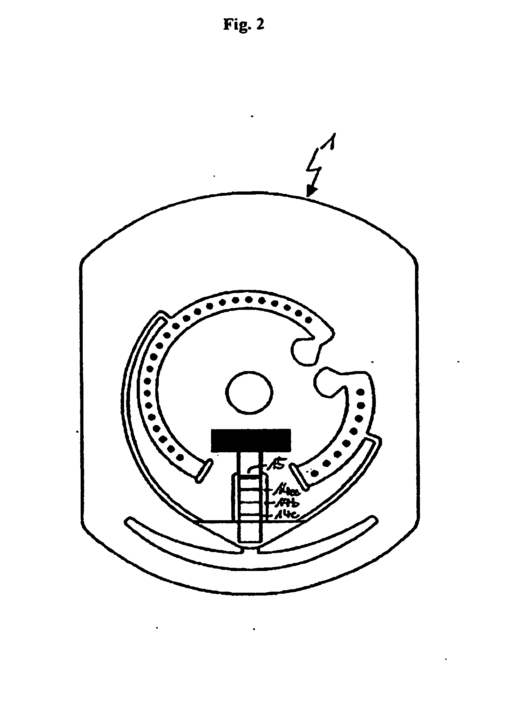

Fig. 2 zeigt die schematische Darstellung einer Aufsicht einer Ausführungsform des erfindungsgemäßen Testelements (1), mit mehreren Analytnachweiszonen (14a) (14b) (14c) ("Paneltest") und einer kombinierten Kontroll- und Kalibrationszone (15).

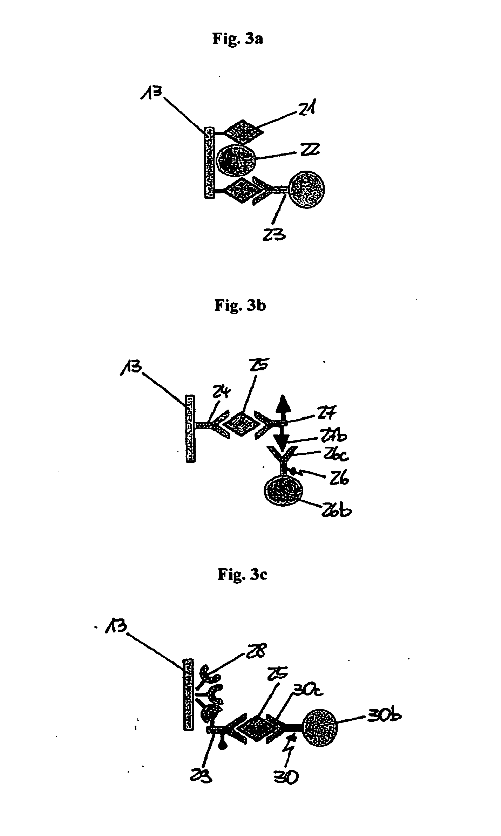

Fig. 3a zeigt eine schematische Darstellung einer Ausführungsform der kombinierten Kontroll- und Kalibrationszone auf einer porösen Matrix (13) mit immobilisiertem g-Globulin-JG9-Latex (22) als Fluorophor und immobilisiertem Troponin T-Polyhapten (21) als Bindepartner für die spezifische Bindung von mit einem Fluorophor markierten Reagenzien, wobei an ein immobilisiertes Troponin T-Polyhapten (21) ein Konjugat aus einem Latex-JG9-Fluorophor und einem Troponin T-spezifischen monoklonalen Antikörper (MAK<TnT>-Lx) (23) als ein mit einem Fluorophor markierten Reagenz gebunden ist. Bei JG9 handelt es sich um ein Quadratsäurederivat.

Fig. 3b zeigt die schematische Darstellung einer Ausführungsform einer Analytnachweiszone, in der die Analyt-spezifischen Reaktanden direkt auf dem Testelement immobilisiert sind. Die Analytnachweiszone besteht aus einem Troponin T-spezifischen monoklonalen Antikörper (MAK<TnT>) (24), der als Analytspezifischer Reaktand direkt auf der porösen Matrix (13) des Testelements immobilisiert ist. Troponin T (25) als Analyt liegt im Sandwich-Komplex mit dem immobilisierten MAK<TnT> (24) und dem mit einem Fluorophor markierten Reagenz vor (26), wobei JG9-Lx (26b) als Fluorophor den Analyten indirekt über einen weiteren Analyt-spezifischen Bindepartner (27) markiert. Bei dem Analyt-spezifischen Bindepartner (27) handelt es sich ein Konjugat mit Digoxigenin (27b) und die indirekte Markierung mit dem mit einem Fluorophor markierten Reagenz wird über die spezifische Wechselwirkung des Anti-Digoxin-Bindepartners (26c) mit dem Digoxigenin (27b) des Analyt-spezifischen Bindepartners (27) vermittelt.

Fig. 3c zeigt die schematische Darstellung einer Ausführungsform einer Analytnachweiszone, in der die Analyt-spezifischen Reaktanden indirekt auf einem Testelement immobilisiert sind. Die Analytnachweiszone besteht aus Streptavidin-Molekülen (28), die direkt auf der porösen Matrix (13) eines Testelements immobilisiert sind. An die Streptavidin-Moleküle (28) ist ein Sandwichkomplex aus einem biotinylierten Troponin T-spezifischen monoklonalen Antikörper (MAK<TnT>-Bi) (29), Troponin T (25) und einem Konjugat aus einem Latex-JG9-Fluorophor und einem Troponin T-spezifischen monoklonalen Antikörper (MAK<TnT>-Lx) (30) gebunden. Der biotinylierte Troponin T-spezifische monoklonale Antikörper (MAK<TnT>-Bi) (29) ist der Analyt-spezifische Reaktand, Troponin T (25) der Analyt, und das Konjugat (30) aus einem Latex-JG9-Fluorophor (30b) und einem Troponin T-spezifischen monoklonalen Antikörper (MAK<TnT>-Lx) (30c) stellt das mit einem Fluorophor markierte Reagenz dar.

**Beispiel 1**

**Herstellung eines Testelements zum Nachweis von Troponin T**

Das Testelement (1) umfasst ein Substrat (2), welches die fluidischen und mikrofluidischen sowie chromatographischen Strukturen enthält. Das Substrat (2) ist durch ein entsprechendes Gegenstück (Deckschicht) abgedeckt (nicht gezeigt in Fig. 1), welches Probenaufgabe und Lüftungsöffnungen enthält, die zu den Strukturen im Substrat (2) korrespondieren. Das Substrat wird mittels Spritzguss aus Polycarbonat (PC) (alternativ ist auch Cyclo-Olefin-Copolymere (COC), Polystyrol (PS), ABS-Kunststoff oder Polymethylmethacrylat (PMMA) als Material möglich) gefertigt (Abmessungen ca. 60 x 80 mm$^2$). Die Oberfläche des Substrats (2), die die fluidischen Strukturen aufweist, kann mittels Plasmabehandlung gereinigt und hydrophilisiert werden. Die Hydrophilisierung der Substratoberfläche kann auch chemisch durch die Verwendung von Detergenzien erfolgen.

Sowohl die Deckschicht als auch das Substrat (2) weisen eine zentrale Aussparung (3) auf, die im Zusammenwirken mit einer entsprechenden Antriebseinheit in einem Messgerät ein Rotieren des scheibenförmigen Testelements (1) ermöglichen. Das Testelement enthält eine Probenzugabeöffnung (4), über die dem Testelement die Probe , insbesondere Vollblut, zugegeben wird. Die Probenzugabeöffnung (4) ist fluidisch mit einem Primärkanalabschnitt (5) verbunden, der den Analyt-spezifischen Reaktanden (6), und die mit Fluorophor markierten Reagenzien (7) enthält. Bei den Analyt-spezifischen Reaktanden (6) handelt es sich hier um einen biotinylierten Troponin T-spezifischen monoklonalen Antikörper (MAK<TnT>-Bi); die mit Fluorophor markierten Reagenzien (7) sind Konjugate aus einem Latex-JG9-Fluorophor und einem Troponin T-spezifischen monoklonalen Antikörper (MAK<TnT>-Lx). Bei JG9 handelt es sich um ein Quadratsäurederivat.

Die Analyt-spezifischen Reaktanden (6), und die mit Fluorophor markierten Reagenzien (7) werden mittels Piezodosierung als Lösung abwechselnd als punktförmige Reagenzienspots in den Primärkanalabschnitt (5) eingebracht und anschließend getrocknet, so dass praktisch seine gesamte innere Fläche mit Reagenzien belegt ist.

Die Reagenzlösungen sind dabei wie folgt zusammengesetzt:
Analyt-spezifische Reaktanden:

| | |
|---|---|
| MES: | 50mM, pH 5.6 |
| RPLA new: | 1% |
| Synperonic P85: | 0.2% |
| Saccharose: | 4% |
| MAK 33: | 3mg/ml |
| MAK33/ Fab' 1-poly: | 2mg/ml |
| MAK<TnT>M11.7-F(ab')2-Bi: | 100μg/ml |

Mit Fluorophor markierte Reagenzien:

| | |
|---|---|
| HEPES | 50mM pH 7.4 |
| PAK<->R-IgG | 1% |
| Synperonic P85 | 0.15% |
| Saccharose | 4% |
| NaCl | 200mM |
| MAK 33 | 3mg/ml |
| MAK33/Fab'1-poly | 2mg/ml |
| MAK<TnT>M7-IgG-Lx(JG9) | 0.35% |

Der Primärkanalabschnitt (5) ist über einen Kapillarstop (9) fluidisch mit einem Sekundärkanalabschnitt (8) verbunden. Der Kapillarstop (9) kann als geometrisches Ventil oder hydrophobe Barriere ausgebildet sein, so dass die durch den Kapillarstop (9) hindurchströmende Probenmenge durch Zentrifugalkräfte mittels der Rotationsgeschwindigkeit des Testelementes (1) gesteuert werden kann. An den Sekundärkanalabschnitt (8) schließen sich die Plasmasammelzone (10) und die Erythrozytensammelzone (11), die als Kammern ausgebildet sind. Bei geeigneten Rotationsgeschwindigkeiten wird im Sekundärkanalabschnitt (8) die Abtrennung roter Blutkörperchen oder anderer zellulärer Probenbestandteile gestartet. Die in dem Primärkanalabschnitt (5) enthaltenen Reagenzien sind beim Eintritt der Probenflüssigkeit in den Sekundärkanalabschnitt (8) bereits gelöst. Bestandteile des Proben-Reagenz-Gemischs werden in den beiden Sammelzonen (10) (11) aufgefangen. Die Plasmasammelzone (10) steht in fluidischer Verbindung mit einer Messkammer (12), die eine poröse Matrix (13) enthält.

Die poröse Matrix (13) (Nitrocellulosemembran auf Kunststoffträgerfolie; 21 x 5 mm$^2$; mit 100 μm PE-Folie verstärkte Cellulosenitrat-Membran (Typ CN 140 von Sartorius, Deutschland)) wird in eine entsprechende Aussparung im Substrat eingebracht und ggf. mittels doppelseitigem Klebeband fixiert. Auf der porösen Matrix (13) befinden sich, jeweils in Form eines Striches, eine Analytnachweiszone (14) und eine kombinierte Kontroll- und Kalibrationszone (15), die vor dem Einbringen der porösen Matrix mittels Strichimprägnierung erzeugt wurden. Hierbei wird die Analytnachweiszone (14) auf die zuvor beschriebene Cellulosenitrat-Membran aufgebracht, indem durch Strichdosierung eine wässrige Streptavidinlösung (4,75 mg/ml) appliziert wird. Die Dosierung wird so gewählt (Dosiermenge 0,12 ml/min, Bahngeschwindigkeit 3 m/min), dass ein Strich mit einer Breite von ca. 0,4 mm entsteht. Dieser Strich dient zum Nachweis des zu bestimmenden Analyts und enthält ca. 0,95 μg Streptavidin pro Membran. Die kombinierte Kontroll- und Kalibrationszone (15) wird in einem Abstand von etwa 4 mm flussabwärts vom Streptavidinstrich erzeugt, indem unter identischen Dosierungsbedingungen ein wässriges Gemisch aus einer Troponin T-Polyhapten-Lösung einer Konzentration von 0,1 mg/ml und aus g-Globulin-JG9-Latex einer Konzentration von 0,03% aufgebracht wird. Diese kombinierte Kontroll- und Kalibrationszone (15) enthält ca. 0,02μg Polyhapten und 0,06 μg g-Globulin-JG9-Latex pro Test.

Nach dem Einbringen der porösen Matrix (13) in das Substrat (2) wird die Abdeckung (Folie oder Spritzgussteil ohne Fluidikstrukturen, die bzw. das ggf. hydrophiliert sein kann) aufgebracht und gegebenenfalls mit dem Substrat (2) permanent verbunden, vorzugsweise verklebt, verschweißt oder verklipst.

Schließlich wird das Substrat gewendet und in die entsprechende Aussparung ein Waste-Vlies (16) (13 x 7 x 1,5 mm$^3$ großes Vlies aus 100 Teilen Glasfaser (Durchmesser 0,49 bis 0,58 μm, Länge 1000 μm) und 5 Teilen Polyvinylalkoholfasern (Kuralon VPB 105-2 von Kuraray) mit einem Flächengewicht von ca.180 g/m$^2$) eingelegt, welches dann mittels eines Klebebandes im Substrat (2) fixiert wird. Das Waste-Vlies (16) liegt auf diese Weise in einer Abfallkammer (17) vor, die in Strömungsrichtung hinter der Messkammer (12) angeordnet ist. In die Abfallkammer (17) können Reaktionsteilnehmer, Proben- und/oder Reagenzbestandteile nach dem Durchströmen der Messkammer (12) entsorgt werden.

Des weiteren kann das Testelement eine Waschlösungszugabeöffnung (18) umfassen, die in fluidischer Verbindung mit einem Waschlösungskanal (19) steht, der wiederum über einen Waschlösungszuführungskanal (20) fluidisch mit der Messkammer (12) verbunden ist. Mit Hilfe dieser Strukturen und geeigneten Zentrifugationsschritten kann Waschlösung in die Messkammer (12) zum Waschen der porösen Matrix (13) geführt werden.

**Beispiel 2**

**Auswertung eines Testelements zum Nachweis von Troponin T**

Testelemente aus Beispiel 1 wurden zum Nachweis von Troponin T in drei verschiedenen Proben mit bekannten Troponin T-Konzentrationen verwendet. Zwei der Proben enthielten Troponin T in einer Konzentration von 0,1 ng/ml und 10 ng/ml in Heparin-Vollblut. Die Herstellung der Proben erfolgte durch die übliche Verdünnung einer Troponin T-Stammlösung von 100 ng/ml die zuvor durch Lösung der erforderlichen Mange eines Troponin T-Lyophilisats der Firma Roche in Humanserum hergestellt wurde. Eine dritte Probe enthielt lediglich Heparin-Vollblut und diente als Negativ-Kontrolle. Für jede Probe wurde ein anderes Testelement gemäß Beispiel 1 eingesetzt.

Der Troponin T-Nachweis erfolgte jeweils unter zwei verschiedenen Auswertebedingungen. Beide Bedingungen unterscheiden sich in ihrem technischen Stand durch die Strahlungsquelle sowie den Eigenschaften der Filter zur Erzeugung der Anregungswellenlänge und der Kamerafilter zur Messung der Emissionswellenlänge für das verwendete Fluorophor (JG9-Latex). In einem Falle handelt es sich um einen Laboraufbau mit einer 100W Halogenlampe; die Filter umfassen einen weiteren Bereich nicht am jeweiligen Maximum des Fluorophors, die sogenannte "Redbox". Im anderen Fall handelt es sich um einen weiterentwickelten Aufbau mit einer 100W Xenonlampe; die Filter umfassen einen engeren Bereich näher am jeweiligen Maximum des Fluorophors, die sogenannte "Linosbox 1". Beide Bedingungen unterscheiden sich stark von den üblichen Variationen zwischen verschiedenen Auswertegeräten, die sich auf die Stärke des jeweils in der Analytnachweiszone gemessenen Analyt-abhängigen Signals auswirken und somit eine Kalibration des Messsignals erfordern, um auf unterschiedlichen Geräten vergleichbare Ergebnisse bei der Analytbestimmung zu erhalten und sollen dadurch die Wirksamkeit der Methode verdeutlichen. Als Auswertegerät selbst wurde die "Linosbox" verwendet.

Vor dem Auftrag der Probe für den eigentlichen Troponin T-Nachweis wurde in der Redbox und der Linosbox 1 nach Anregung mit Strahlung einer Anregungswellenlänge von 633nm jeweils das Signal der kombinierten Kontroll- und Kalibrationszone auf dem Testelement bestimmt ("Trockenmessung", siehe Tabelle 1). Die absoluten Signalstärken unterscheiden sich zwischen Redbox und Linosbox 1 zwar in den absoluten Werten, ergaben jedoch im Mittel erwartungsgemäß keine signifikanten Schwankungen zwischen den einzelnen Proben (siehe Tabelle 1, Zeile "Mittelwert").

Tabelle 1:

Bestimmung des Signals in der Analytnachweiszone vor Applikation der Probe auf das Testelement ("Trockenmessung")

## Redbox

**Auswertung kombinierte Kontroll- und Kalibrationszone**

| Membran Nr. | | 29 (6/9/9) | |
|---|---|---|---|
| Konz. TnT [ng/ml] | 0 | 0,1 | 10 |
| Anzahl [n] | 5 | 5 | 5 |
| Mittelwert | 2044 | 2032 | 2044 |
| Standardabweichung | 18,4 | 54,3 | 42,6 |
| VK | 0,9% | 2,7% | 2,1% |

## Linosbox1

**Auswertung kombinierte Kontroll- und Kalibrationszone**

| Membran Nr. | | 29 (6/9/9) | |
|---|---|---|---|
| Konz. TnT [ng/ml] | 0 | 0,1 | 10 |
| Anzahl [n] | 5 | 5 | 5 |
| Mittelwert | 53988 | 53945 | 54044 |
| Standardabweichung | 553,1 | 1248,5 | 969,6 |
| VK | 1,0% | 2,3% | 1,8% |

Konz. TnT [ng/ml]: Konzentration von Troponin T in der Probe, die nach der Trockenmessung auf dem gleichen Testträger analysiert wurde.
Anzahl [n]: Anzahl der durchgeführten Messungen.
VK: Variationskoeffizient; Standardabweichung/ Mittelwert in Prozent.

Anschließend erfolgte mit den drei Proben die Messung des Signals in der Analytnachweiszone jeweils unter den Bedingungen der Redbox und der Linosbox1. Hierzu wurden jeweils 40 µl in die Probenaufgabeöffnung (4) des Testelements pipettiert und durch Kapillarkräfte in den Primärkanalabschnitt (5) eingezogen. Nachdem die im Primärkanalabschnitt (5) vorhandenen Reagenzien (6) (7) durch die Probe nach Inkubation gelöst waren, wurde die Lösung durch Zentrifugation in die Messkammer (12) mit der porösen Matrix (13) überführt und die festen Blutbestandteile in die daran anschließende Erythrozytensammelzone (11) separiert. Durch Reduzierung der Zentrifugalkraft wurde das entstandene Plasma-/Reagenzgemisch kapillarisch über die poröse Matrix (13) chromatographiert, wo der Analyt dann in der Analytnachweiszone (14) immobilisiert wurde. Durch Waschen mit Waschpuffer wurde der überschüssige Fluorophor auf dem gleichen Weg vor der Messung aus der Membran entfernt.

Das Signal in der Analytnachweiszone wurde in der Redbox und der Linosbox 1 nach Anregung mit Strahlung einer Anregungswellenlänge von 633 nm bestimmt (siehe "Mittelwert" in Tabelle 2). Erwartungsgemäß sind die Signalstärken der Troponin T-enthaltenden Proben aufgrund der Analyt-Abhängigkeit des Signals signifikant gegenüber dem Hintergrundsignal der Negativkontrolle (Konz. TnT 0 ng/ml) erhöht. Auch die Signalstärken zwischen den Proben entsprechen in ihrer Relation der eingesetzten Troponin T-Konzentration, d.h. ca. 100-fach stärkeres Signal bei 100-fach höherer Konzentration. Ein Vergleich der Absolutwerte zwischen der Redbox- und der Linosbox1-Versuchsbedingung zeigt jedoch eine Abweichung mit einem Faktor von mehr als 20 (Vergleiche Zeile "Mittelwert" Redbox und Zeile "Mittelwert" Linosbox1 in Tabelle 2). Erst durch das erfindungsgemäße In Beziehung-Setzen des zuvor in der kombinierten Kontroll- und Kalibrationszone bestimmten Signals mit dem in der Analytnachweiszone bestimmten Signal führt zu einem kalibrierten Messsignal, das sich nur noch geringfügig zwischen den beiden Versuchsbedingungen unterscheidet (siehe Tabelle 2, Zeile "Korrektur").

## Tabelle 2:

### Bestimmung des Signals in der Analytnachweiszone nach Applikation der Probe auf das Testelement

### Redbox

**Auswertung Analytnachweiszone**

| Membran Nr. | 29 (6/9/9) | | |
|---|---|---|---|
| Konz. TnT [ng/ml] | 0 | 0,1 | 10 |
| Anzahl [n] | 5 | 5 | 5 |
| Mittelwert | 18 | 101 | 10612 |
| Standardabweichung | 2,1 | 3,2 | 178,9 |
| VK | 11,9% | 3,2% | 1,7% |

| Korrektur | 18/2044 $=8,8.10^{-3}$ | 101/2032 $=49,7.10^{-3}$ | 10612/2044 $=5,19$ |
|---|---|---|---|

### Linosbox1

**Auswertung Analytnachweiszone**

| Membran Nr. | 29 (6/9/9) | | |
|---|---|---|---|
| Konz. TnT [ng/ml] | 0 | 0,1 | 10 |
| Anzahl [n] | 5 | 7 | 5 |
| Mittelwert | 361 | 2146 | 249298 |
| Standardabweichung | 27 | 71 | 5310 |
| VK | 7,4% | 3,3% | 2,1% |

| Korrektur | 361/53968 $=6,7.10^{-3}$ | 2146/53945 $=39,7.10^{-3}$ | 249298/54044 $=4,61$ |
|---|---|---|---|

Konz. TnT [ng/ml]: Konzentration von Troponin T in der Probe.
Anzahl [n]: Anzahl der durchgeführten Messungen.
VK: Variationskoeffizient; Standardabweichung/ Mittelwert in Prozent.

**Patentansprüche**

1. Testelement, insbesondere in Form eines nach dem Sandwichprinzip arbeitenden immunologischen Teststreifens, für den fluorophoren Nachweis eines oder mehrerer Analyten in einer Probe, umfassend

   - eine Analytnachweiszone, und
   - eine kombinierte Kontroll- und Kalibrationszone,
   **dadurch gekennzeichnet, dass**
   die kombinierte Kontroll- und Kalibrationszone

   a) Fluorophor umfasst, wobei der auf der kombinierten Kalibrations-/Kontrollzone gebundene Fluorophor als Kalbrationsstandard dient und
   b) Bindepartner für die spezifische Bindung von mit einem Fluorophor markierten Reagenzien umfasst, wobei die Bindepartner für die spezifische Bindung von mit einem Fluorophor markierten Reagenzien immobilisiert vorliegen, wobei die mit einem Fluorophor markierten Reagenzien mit dem Analyten spezifisch durch direkte oder indirekte Bindung wechselwirken.

2. Testelement gemäß Anspruch 1, **dadurch gekennzeichnet, dass** die Analytnachweiszone und/oder die kombinierte Kontroll- und Kalibrationszone in Form eines Striches oder eines im Wesentlichen runden Spots vorliegen.

3. Testelement gemäß einem der Ansprüche 1 bis 2, **dadurch gekennzeichnet, dass** das Testelement ein immunologisches Testelement ist, insbesondere in Form eines immunologischen Chromatographieteststreifens.

4. Testelement gemäß einem der Ansprüche 1 bis 3, **dadurch gekennzeichnet, dass** das Testelement ein Microarray ist.

5. Testelement gemäß einem der vorangehenden Ansprüche, **dadurch gekennzeichnet, dass** in der kombinierten Kontroll- und Kalibrationszone die Menge des Fluorophors zwischen 0,001 $\mu$g und 5$\mu$g beträgt und die Menge der mit einem Fluorophor markierten Reagenzien zwischen 1ng und 500ng beträgt.

6. Verfahren zur Kalibration eines Analyt-spezifischen Messsignals unter Verwendung des Testelements gemäß einem der Ansprüche 1 bis 5 und eines für die Auswertung des Testelements geeigneten Analysegeräts, umfassend die folgenden Schritte:

   a) Erzeugung eines Signals in der kombinierten Kontroll- und Kalibrationszone durch Anregung des Fluorophors in der kombinierten Kontroll- und Kalibrationszone in Abwesenheit der mit einem Fluorophor markierten Reagenzien,
   b) Messung des in Schritt a) erzeugten Signals,
   c) in Kontakt bringen der Probe mit dem Testelement und mit den mit einem Fluorophor markierten Reagenzien, so dass der der Analyt - sofern in der Probe vorhanden - zu einem in der Analytnachweiszone detektierbaren Signal führt;
   d) Erzeugung eines Signals in der Analynachweiszone durch Anregung des Fluorophors der in Anwesenheit des Analyten in der Analytnachweiszone vorhandenen mit einem Fluorophor markierten Reagenzien,
   e) Messung des in Schritt d) erzeugten Signals,
   f) In Beziehung setzen der in Schritt b) und e) gemessenen Signale.

7. Verfahren zur Bestimmung der Konzentration eines Analyten in einer Probe unter Verwendung des Testelements gemäß einem der Ansprüche 1 bis 5 und eines für die Auswertung des erfindungsgemäßen Testelements geeigneten Analysegeräts, umfassend die folgenden Schritte:

   a) Erzeugung eines Signals in der kombinierten Kontroll- und Kalibrationszone durch Anregung des Fluorophors in der kombinierten Kontroll- und Kalibrationszone in Abwesenheit der mit einem Fluorophor markierten Reagenzien,
   b) Messung des in Schritt a) erzeugten Signals,
   c) in Kontakt bringen der Probe mit dem Testelement und mit den mit einem Fluorophor markierten Reagenzien, so dass der der Analyt - sofern in der Probe vorhanden - zu einem in der Analytnachweiszone detektierbaren Signal führt;
   d) Erzeugung eines Signals in der Analynachweiszone durch Anregung des Fluorophors der in Anwesenheit des Analyten in der Analytnachweiszone vorhandenen mit einem Fluorophor markierten Reagenzien,
e) Messung des in Schritt d) erzeugten Signals,
f) In Beziehung setzen der in Schritt b) und e) gemessenen Signale.
g) Vergleich der Beziehung aus Schritt f) mit einer Eichkurve,
h) Bestimmung der Konzentration des Analyten aufgrund des in Schritt g) erfolgten Vergleiches.

8. Verfahren gemäß Anspruch 6 oder 7, **dadurch gekennzeichnet, dass** die in Schritt b) und e) gemessenen Signale miteinander verrechnet werden.

9. Verfahren gemäß einem der Ansprüche 6 bis 8, **dadurch gekennzeichnet, dass** die Erzeugung eines Signals in der kombinierten Kontroll- und Kalibrationszone durch Anregung des Fluorophors in der kombinierten Kontroll- und Kalibrationszone in Abwesenheit der mit einem Fluorophor markierten Reagenzien (Schritt a)) und die Messung des durch den Fluorophor erzeugten Signals (Schritt b)) erfolgen, bevor die Probe mit dem Testelement und mit den mit einem Fluorophor markierten Reagenzien in Kontakt gebracht wird (Schritt c)).

10. Verwendung des Testelements gemäß einem der Ansprüche 1 bis 5 zum Kalibrieren eines in der Analytnachweiszone eines Testelements erzeugten Signals.

**Claims**

1. Test element, more particularly in the form of an immunological test strip operating according to the sandwich principle, for the fluorophoric detection of one or more analytes in a sample, comprising

    - an analyte detection zone, and
    - a combined control and calibration zone, **characterized in that**
    the combined control and calibration zone

    a) comprises fluorophore, wherein the fluorophore bound on the combined calibration/control zone serves as calibration standard and
    b) comprises binding partners for the specific binding of reagents labelled with a fluorophore, wherein the binding partners for the specific binding of reagents labelled with a fluorophore are present in an immobilized state, wherein the reagents labelled with a fluorophore interact with the analyte specifically by means of direct or indirect binding.

2. Test element according to Claim 1, **characterized in that** the analyte detection zone and/or the combined control and calibration zone are present in the form of a line or of a substantially round spot.

3. Test element according to either of Claims 1 to 2, **characterized in that** the test element is an immunological test element, more particularly in the form of an immunological chromatography test strip.

4. Test element according to any of Claims 1 to 3, **characterized in that** the test element is a microarray.

5. Test element according to any of the preceding claims, **characterized in that**, in the combined control and calibration zone, the amount of fluorophore is between 0.001 $\mu$g and 5 $\mu$g and the amount of reagents labelled with a fluorophore is between 1 ng and 500 ng.

6. Method for calibrating an analyte-specific measurement signal using the test element according to any of Claims 1 to 5 and an analyser suitable for evaluating the test element, comprising the following steps:

    a) generating a signal in the combined control and calibration zone by exciting the fluorophore in the combined control and calibration zone in the absence of the reagents labelled with a fluorophore,
    b) measuring the signal generated in step a),
    c) contacting the sample with the test element and with the reagents labelled with a fluorophore, and so the analyte - if present in the sample - leads to a signal detectable in the analyte detection zone;
    d) generating a signal in the analyte detection zone by exciting the fluorophore of the reagents labelled with a fluorophore that are in the presence of the analyte in the analyte detection zone,
    e) measuring the signal generated in step d),

f) relating the signals measured in steps b) and e).

7. Method for determining the concentration of an analyte in a sample using the test element according to any of Claims 1 to 5 and an analyser suitable for evaluating the test element according to the invention, comprising the following steps:

a) generating a signal in the combined control and calibration zone by exciting the fluorophore in the combined control and calibration zone in the absence of the reagents labelled with a fluorophore,
b) measuring the signal generated in step a),
c) contacting the sample with the test element and with the reagents labelled with a fluorophore, and so the analyte - if present in the sample - leads to a signal detectable in the analyte detection zone;
d) generating a signal in the analyte detection zone by exciting the fluorophore of the reagents labelled with a fluorophore that are in the presence of the analyte in the analyte detection zone,
e) measuring the signal generated in step d),
f) relating the signals measured in steps b) and e),
g) comparing the relationship from step f) with a calibration curve,
h) determining the concentration of the analyte on the basis of the comparison performed in step g).

8. Method according to Claim 6 or 7, **characterized in that** the signals measured in steps b) and e) are used together in a calculation.

9. Method according to any of Claims 6 to 8, **characterized in that** the generation of a signal in the combined control and calibration zone by exciting the fluorophore in the combined control and calibration zone in the absence of the reagents labelled with a fluorophore (step a)) and the measurement of the signal generated by the fluorophore (step b)) take place before the sample is contacted with the test element and with the reagents labelled with a fluorophore (step c)).

10. Use of the test element according to any of Claims 1 to 5 for calibrating a signal generated in the analyte detection zone of a test element.

**Revendications**

1. Elément de test, en particulier en forme d'une bande de test immunologique opérant suivant le principe du sandwich, pour la détection fluorophore d'un ou de plusieurs analyte(s) dans un échantillon, comprenant:

- une zone de détection d'analyte, et
- une zone combinée de contrôle et de calibrage,
**caractérisé en ce que** la zone combinée de contrôle et de calibrage

a) comprend un fluorophore, dans lequel le fluorophore lié à la zone combinée de contrôle et de calibrage sert de standard de calibrage et
b) comprend des partenaires de liaison pour la liaison spécifique de réactifs marqués avec un fluorophore, dans lequel les partenaires de liaison pour la liaison spécifique de réactifs marqués par un fluorophore sont immobilisés, dans lequel les réactifs marqués par un fluorophore interagissent avec l'analyte spécifiquement par liaison directe ou indirecte.

2. Elément de test selon la revendication 1, **caractérisé en ce que** la zone de détection d'analyte et/ou la zone combinée de contrôle et de calibrage se présentent sous la forme d'un trait ou d'une tache essentiellement ronde.

3. Elément de test selon l'une des revendications 1 à 2, **caractérisé en ce que** l'élément de test est un élément de test immunologique, en particulier sous la forme d'une bande de test de chromatographie immunologique.

4. Elément de test selon l'une quelconque des revendications 1 à 3, **caractérisé en ce que** l'élément de test est une biopuce.

5. Elément de test selon l'une quelconque des revendications précédentes, **caractérisé en ce que**, dans la zone combinée de contrôle et de calibrage, la quantité de fluorophore vaut entre 0,001 $\mu$g et 5 $\mu$g et la quantité des réactifs marqués avec un fluorophore vaut entre 1 ng et 500 ng.

**6.** Procédé de calibrage d'un signal de mesure spécifique d'analyte avec utilisation de l'élément de test selon l'une quelconque des revendications 1 à 5 et d'un appareil d'analyse convenant pour l'exploitation de l'élément de test, comprenant les étapes suivantes:

a) production d'un signal dans la zone combinée de contrôle et de calibrage par excitation du fluorophore dans la zone de contrôle et de calibrage en l'absence des réactifs marqués avec un fluorophore,
b) mesure du signal produit à l'étape a),
c) mise en contact de l'échantillon avec l'élément de test et avec les réactifs marqués par un fluorophore, de telle manière que l'analyte - dans la mesure où il est présent dans l'échantillon - conduise à un signal détectable dans la zone de détection d'analyte;
d) production d'un signal dans la zone de détection d'analyte par excitation du fluorophore des réactifs marqués avec un fluorophore présents dans la zone de détection d'analyte en présence de l'analyte,
e) mesure du signal produit à l'étape d),
f) mise en relation des signaux mesurés à l'étape b) et à l'étape e).

**7.** Procédé de détermination de la concentration d'un analyte dans un échantillon avec utilisation de l'élément de test selon l'une quelconque des revendications 1 à 5 et d'un appareil d'analyse convenant pour l'exploitation de l'élément de test selon l'invention, comprenant les étapes suivantes:

a) production d'un signal dans la zone combinée de contrôle et de calibrage par excitation du fluorophore dans la zone combinée de contrôle et de calibrage en l'absence des réactifs marqués avec un fluorophore,
b) mesure du signal produit à l'étape a),
c) mise en contact de l'échantillon avec l'élément de test et avec les réactifs marqués avec un fluorophore, de telle manière que l'analyte - dans la mesure où il est présent dans l'échantillon - conduise à un signal détectable dans la zone de détection d'analyte;
d) production d'un signal dans la zone de détection d'analyte par excitation du fluorophore des réactifs marqués par un fluorophore présents dans la zone de détection d'analyte en présence de l'analyte,
e) mesure du signal produit à l'étape d),
f) mise en relation des signaux mesurés aux étapes b) et e),
g) comparaison de la relation de l'étape f) avec une courbe d'étalonnage,
h) détermination de la concentration de l'analyte sur la base de la comparaison effectuée à l'étape g).

**8.** Procédé selon la revendication 6 ou 7, **caractérisé en ce que** l'on compense mutuellement les signaux mesurés aux étapes b) et e).

**9.** Procédé selon l'une quelconque des revendications 6 à 8, **caractérisé en ce que** l'on effectue la production d'un signal dans la zone combinée de contrôle et de calibrage par excitation du fluorophore dans la zone combinée de contrôle et de calibrage en l'absence des réactifs marqués avec un fluorophore (étape a)) et la mesure du signal produit par le fluorophore (étape b)), avant que l'échantillon soit mis en contact avec l'élément de test et avec les réactifs marqués avec un fluorophore (étape c)).

**10.** Utilisation de l'élément de test selon l'une quelconque des revendications 1 à 5 pour le calibrage d'un signal produit dans la zone de détection d'analyte d'un élément de test.

Fig. 1

Fig. 2

**Fig. 3a**

**Fig. 3b**

**Fig. 3c**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2007042219 A
- EP 1412533 A
- WO 2008105814 A
- US 20040126767 A
- US 4168146 A, Grubb
- US 4366241 A, Tom
- US 4235601 A, Deutsch
- US 4442204 A, Liotta
- US 5208535 A, Buechler
- WO 9706439 A
- EP 0291194 A
- US 5591645 A
- US 4861711 A
- US 5141850 A
- US 6506612 B
- US 5458852 A
- US 5073484 A
- US 20070048807 A1